# EUROPÄISCHE PATENTSCHRIFT

(11) **EP 2 300 149 B1**
(45) Veröffentlichungstag und Bekanntmachung des Hinweises auf die Patenterteilung: **12.12.2012**
(21) Anmeldenummer: 09779773.2
(22) Anmeldetag: 16.06.2009
(51) Int. Cl.: B01D 1/22, C08G 18/10, C08G 18/48, C08G 18/75, C08G 18/82, C08G 18/08, C07C 263/18, C07C 263/20, B01J 19/18

(54) **VERFAHREN ZUR ENTFERNUNG VON NICHT UMGESETZTEN ISOCYANAT AUS DESSEN UMSETZUNGSPRODUKT**
METHOD FOR REMOVING NON-REACTED ISOCYANATE FROM ITS REACTION PRODUCT
PROCÉDÉ SERVANT À ÉLIMINER L'ISOCYANATE NON DÉCOMPOSÉ DE SON PRODUIT DE DÉCOMPOSITION

(30) Priorität: 08.07.2008 EP 08104668
(43) Veröffentlichungstag der Anmeldung: 30.03.2011
(73) Patentinhaber: Construction Research & Technology GmbH, 83308 Trostberg (DE)
(72) Erfinder: KLAPDOHR, Simone, 83022 Rosenheim (DE); WALTHER, Burkhard, 84518 Garching (DE); MACK, Helmut, 83278 Traunstein (DE); CAI, Zhizhong, 83308 Trostberg (DE); MARC, Laurent, F-77850 Hericy (FR); MEZGER, Jochen, 84518 Garching an der Alz (DE); AUSTERMANN, Tobias, 48153 Münster (DE); FLAKUS, Silke, 85560 Ebersberg (DE)
(86) Internationale Anmeldenummer: PCT/EP2009/057414
(87) Internationale Veröffentlichungsnummer: WO 2010/003770

(56) Entgegenhaltungen:
- DE-A1-102007 051 274
- GB-A- 2 416 500
- US-A1- 2005 145 474
- JACHUCK R J ET AL: "PROCESS INTENSIFICATION: THE OPPORTUNITY PRESENTED BY SPINNING DISC REACTOR TECHNOLOGY" 1. Januar 1997 (1997-01-01), INSTITUTION CHEMICAL ENGINEERS. SYMPOSIUM SERIES, XX, XX, PAGE(S) 417 - 424 , XP001247804 Seite 421 - Seite 422

## Beschreibung

Die vorliegende Erfindung betrifft ein Verfahren zur Entfernung von Isocyanat aus einem Umsetzungsprodukt von Isocyanat mit gegenüber Isocyanaten reaktiven Verbindungen.

Isocyanate sind wertvolle Rohstoffe und werden beispielsweise für die Herstellung von Polyurethanen und Polyharnstoffen eingesetzt. Hierzu werden die Isocyanate mit Polyalkoholen bzw. Polyaminen umgesetzt. Die erhaltenen Produkte spielen beispielsweise in der industriellen Herstellung von Chemikalien, Klebstoffen, Kunststoffen und Farben eine wichtige Rolle. Bedingt durch die bei der Herstellung entstehende Molmassenverteilung enthalten die Umsetzungsprodukte jedoch häufig noch Mengen an nicht umgesetzten monomeren Isocyanaten oder niedermolekulare Umsetzungsprodukte, welche Isocyanatgruppen aufweisen. Diese können aus den Umsetzungsprodukten ausgasen und als reizende, sensibilisierende oder toxische Stoffe eine Gesundheitsbelastung für den Verarbeiter und Endkunden darstellen. Weiterhin können die im Produkt verbleibenden monomeren Isocyanate oder niedermolekularen Umsetzungsprodukte die Produkteigenschaften unvorteilhaft beeinflussen.

Eine bekannte Methode monomeres Diisocyanat und niedermolekulare Umsetzungsprodukte aus Reaktionsgemischen zu entfernen ist eine Destillation. So ist beispielsweise aus der EP 105242A2 bekannt, den verbleibenden Monomergehalt eines Umsetzungsproduktes von Isocyanat durch Destillation mit Hilfe eines Dünnschichtverdampfers zu reduzieren, wobei das Umsetzungsprodukt zunächst mit einem inerten Lösungsmittel verdünnt wird. Dies hat jedoch den Nachteil, dass zumindest ein Teil des inerten Lösungsmittels im Produkt verbleibt und bei den folgenden Anwendungen zu Problemen führen kann. Bei Produktwechseln ist außerdem eine aufwändige Reinigung der Apparatur notwendig. Weiterhin ist diese Methode nach dem Stand der Technik apparativ aufwändig und deshalb teuer.

Aufgabe der vorliegenden Erfindung war es deshalb, ein prozessual flexibles und wirtschaftliches Verfahren zur Entfernung von Isocyanat aus einem Umsetzungsprodukt von Isocyanat mit gegenüber Isocyanaten reaktiven Verbindungen bereit zu stellen. Das Verfahren sollte in einfacher Weise durchführbar sein und eine gute und reproduzierbare Produktqualität gewährleisten. Weiterhin sollten auch hochviskose Umsetzungsprodukte ohne den Zusatz eines inerten Lösungsmittels aufgereinigt werden können.

Die Lösung dieser Aufgabe ist ein Verfahren zur Entfernung von Isocyanat aus einem Umsetzungsprodukt von Isocyanat mit gegenüber Isocyanaten reaktiven Verbindungen, wobei man das Umsetzungsprodukt auf die Oberfläche eines rotierenden Körpers A aufträgt, wobei das Umsetzungsprodukt über die Oberfläche des rotierenden Körpers A zu einem äußeren Bereich der Oberfläche des rotierenden Körpers A fließt und dabei Isocyanat, das zur Herstellung des Umsetzungsproduktes eingesetzt und nicht umgesetzt wurde, aus der Mischung verdampft.

Der rotierende Köper A kann scheiben-, vasen-, ring- oder kegelförmig ausgeführt werden, wobei eine waagrechte oder eine von der Waagrechten um bis zu 45°C abweichende Drehscheibe als bevorzugt anzusehen ist. Normalerweise weist der Körper A einen Durchmesser von 0,10 m bis 3,0 m, bevorzugt 0,20 m bis 2,0 m und besonders bevorzugt von 0,20 m bis 1,0 m auf. Die Oberfläche kann glatt sein oder beispielsweise riffel- oder spiralförmige Einformungen aufweisen, welche Einfluss auf die Verweilzeit des Reaktionsgemischs ausüben. Zweckmäßigerweise ist der Körper A in einem bezüglich den Bedingungen des erfindungsgemäßen Verfahrens beständigen Container eingebaut.

Die Drehgeschwindigkeit des Körpers A sowie die Dosierungsrate des Umsetzungsproduktes sind variabel. Üblicherweise beträgt die Umdrehungsgeschwindigkeit in Umdrehungen pro Minute 1 bis 20000, bevorzugt 100 bis 5000 und besonders bevorzugt 500 bis 3000. Das Volumen des Umsetzungsproduktes, welches sich pro Flächeneinheit der heißen Oberfläche auf dem rotierenden Körper A befindet, beträgt typischerweise 0,03 bis 40 mL/dm², bevorzugt 0,1 bis 10 mL/dm², besonders bevorzugt 1,0 bis 5,0 mL/dm². Die mittlere Verweilzeit (Häufigkeitsmittel des Verweilzeitspektrums) der Mischung ist unter anderem von derGröße der Oberfläche, von der Art des Umsetzungsproduktes und dem enthaltenen Isocyanat, von der Temperatur der Oberfläche sowie von der Umdrehungsgeschwindigkeit des rotierenden Körpers A abhängig und beträgt normalerweise zwischen 0,01 und 60 Sekunden, besonders bevorzugt zwischen 0,1 und 10 Sekunden, insbesondere 1 bis 7 Sekunden und ist somit als ausgesprochen kurz anzusehen. Dies gewährleistet, dass das Ausmaß von möglichen Zersetzungsreaktionen und die Bildung unerwünschter Produkte stark reduziert werden und somit die Qualität der Substrate erhalten bleibt.

In einer bevorzugten Ausführungsform der Erfindung wird die Entfernung des lsocyanates mittels einer Apparatur durchführt, die
- α): einen um eine bevorzugt zentral angeordnete Rotationsachse rotierenden Körper A und
- β): ein Dosierungssystem,
aufweist.

In einer weiteren Ausführungsform kann die Apparatur eine Quenscheinrichtung aufweisen. Die Quenscheinrichtung liegt bevorzugt als mindestens eine die Drehscheibe umgebende Kühlwand vor, auf die das Umsetzungsprodukt nach Verlassen der Oberfläche trifft. In dieser Ausführungsform gewährleistet das erfindungsgemäße Verfahren, dass das Umsetzungsprodukt, aus welchem das Isocyanat entfernt werden soll, durch den Körper A in sehr kurzer Zeit stark erwärmt werden kann, wobei durch das nachfolgende Quenschen thermisch bedingte, unerwünschte Nebenreaktionen verhindert werden. Die abrupte Abkühlung mittels der Quenscheinrichtung erfolgt innerhalb von maximal fünf Sekunden, bevorzugt innerhalb von nur einer Sekunde.

Zu einer effektiven Entfernung des Isocyanates kann es auch zweckmäßig sein, das Umsetzungsprodukt mehrfach über die Oberfläche des rotierenden Körpers A zu leiten. In einer weiteren Ausführungsform der Erfindung erstreckt sich die Oberfläche auf weitere rotierende Körper, so dass das Umsetzungsprodukt von der Oberfläche des rotierenden Körpers A auf die Oberfläche mindestens eines weiteren rotierenden Körpers gelangt. Die weiteren rotierenden Körper sind zweckmäßigerweise entsprechend dem Körper A beschaffen. Typischerweise füttert dann Körper A die weiteren Körper mit dem Umsetzungsprodukt. Das Umsetzungsprodukt verlässt diesen mindestens einen weiteren Körper, und kann dann bei Bedarf abrupt mittels der Quenscheinrichtung abgekühlt werden.

Es ist als bevorzugt anzusehen, dass das Umsetzungsprodukt auf der Oberfläche des rotierenden Körpers A in Form eines Films vorliegt, der eine durchschnittliche Dicke zwischen 0,1 µm und 6,0 mm, bevorzugt zwischen 60 und 1000 µm, besonders bevorzugt zwischen 100 und 500 µm aufweist.

Das erfindungsgemäße Verfahren kann bei Normaldruck oder leichtem Überdruck und auch in einer Atmosphäre von trockenem Schutzgas durchgeführt werden. Es kann aber auch zweckmäßig sein, ein Vakuum zu erzeugen, wobei sich insgesamt Drücke zwischen 0,001 mbar und 1100 mbar, besonders bevorzugt zwischen 0,01 mbar und 40 mbar, insbesondere zwischen 0,02 mbar und 20 mbar als vorteilhaft erwiesen haben. Eine bevorzugte Ausführungsform der vorliegenden Erfindung sieht weiterhin vor, dass das verdampfte Isocyanat mit einem Gas oder trockener Luft, insbesondere Inertgas ausgetrieben wird. Es ist weiterhin als bevorzugt anzusehen, dass gleichzeitig ein Vakuum angelegt wird und das verdampfte Isocyanat mit einem Gas oder trockener Luft, insbesondere Inertgas ausgetrieben wird.

Die Temperatur des rotierenden Körpers A, insbesondere der der Mischung zugewandten Oberfläche, kann in weiten Bereichen variiert werden und hängt sowohl von den eingesetzten Umsetzungsprodukten, dem Isocyanat, der Verweilzeit auf dem Körper A als auch vom Druck ab. Es haben sich Temperaturen zwischen 70 und 300 °C, besonders bevorzugt zwischen 25 und 270 °C, insbesondere zwischen 150 und 250 °C als zweckmäßig erwiesen. Der rotierende Körper A und/oder das Umsetzungsprodukt kann beispielsweise elektrisch, mit einer Wärmeträgerflüssigkeit, mit Dampf, mit einem Laser, mit Mikrowellenstrahlung oder mittels Infrarotstrahlung beheizt werden.

Es hat sich weiterhin als zweckmäßig erwiesen, das verdampfte Isocyanat an einem Körper mit einer Temperatur zwischen -196 °C und 120 °C, besonders bevorzugt zwischen -78 und 20 °C, insbesondere zwischen -78 und 0 °C zu kondensieren. In diesem Zusammenhang sieht eine bevorzugte Ausführungsform vor, dass der rotierende Körper A von mindestens einer Oberfläche umgeben ist, an welcher Isocyanat kondensieren kann, wobei es bevorzugt ist, dass die Oberfläche eine Neigung aufweist, so dass das kondensierte Isocyanat durch Gravitation entlang der Oberfläche von dem rotierenden Körper A weggeführt wird.

Es kann aber auch zweckmäßig sein, die den Körper A umgebenden Oberflächen zu beheizen, um eine Kondensation von Isocyanat zu verhindern. Das verdampfte Isocyanat kann in dieser Ausführungsform durch ein Vakuum oder einen Inertgasstrom abgeführt werden.

Der Isocyanat-Gehalt der eingesetzten Umsetzungsprodukte ist nicht kritisch. Insbesondere eignet sich das erfindungsgemäße Verfahren, wenn der Isocyanat-Gehalt der eingesetzten Umsetzungsprodukte direkt vor dem Auftragen auf die Oberfläche des rotierenden Körpers A zwischen 0,01 und 95 Gew.-%, besonders bevorzugt zwischen 0,1 und 75 Gew.-%, insbesondere zwischen 0,2 und 67 Gew.-% beträgt, bezogen auf das Gesamtgewicht des Umsetzungsproduktes. Es ist hierbei als bevorzugt anzusehen, dass der Isocyanat-Gehalt in der Mischung nach dem Entfernen des Isocyanates durch Verdampfen auf der Oberfläche des rotierenden Körpers A zwischen 0,001 und 10 Gew.-%, besonders bevorzugt zwischen 0,02 und 5 Gew.-%, insbesondere zwischen 0,05 und 2 Gew.-% beträgt, bezogen auf das Gesamtgewicht des Umsetzungsproduktes.

Bei Isocyanat handelt es sich bevorzugt um eine aliphatische, cycloaliphatische, araliphatische und/oder aromatische Verbindung, bevorzugt um ein Diisocyanat oder Triisocyanat, wobei es sich auch um Mischungen dieser Verbindungen handeln kann. Hierbei ist es als bevorzugt anzusehen, dass das Umsetzungsprodukt auf der Umsetzung von Hexamethylendiisocyanat-1,6 (HDI), 1-Isocyanato-3,3,5-trimethyl-5-isocyanatomethylcyclohexan (IPDI), 2,4- und/oder 2,6-Toluylendiisocyanat (TDI) und/oder 4,4' -, 2,4' - und/oder 2,2' -Diphenylmethandiisocyanat (MDI), m-Xylendiisocyanat (MXDI), m- oder p-Tetramethylxylendiisocyanat (m-TMXDI, p-TMXDI), 4,4'-Dicyclohexylmethandiisocyanat (H12MDI), Naphthalin-1,5-Diisocyanat, Cyclohexan-1,4-diisocyanat, hydriertes Xylylen-diisocyanat-(H6XDI), 1-Methyl-2,4-diisocyanato-cyclohexan, Tetramethoxybutan-1,4-diisocyanat, Butan-1,4-diisocyanat, 1,6-Diisocyanato-2,2,4-trimethylhexan, 1,6-Diisocyanato-2,4,4-trimethylhexan, 1-Isocyanato-1-methyl-4(3)-isocyanatamethylcyclohexan (IMCI) sowie 1,12-Dodecandiisocyanat (C12DI) mit gegenüber Isocyanaten reaktiven Verbindungen basiert.

Die vorliegende Erfindung sieht vor, dass bevorzugt Hexamethylendiisocyanat-1,16 (HDI), 1-Isocyanato-3,3,5-trimethyl-5-isocyanatomethylcyclohexan (IPDI), 2,4-und/oder 2,6-Toluylendiisocyanat (TDI) und/oder 4,4' -, 2,4' - und/oder 2,2' - Diphenylmethandiisocyanat (MDI), m-Xylendiisocyanat (MXDI), m- oder p-Tetramethylxylendiisocyanat (m-TMXDI, p-TMXDI), 4,4'-Dicyclohexylmethandiisocyanat (H12MDI), Naphthalin-1,5-Diisocyanat, Cyclohexan-1,4-diisocyanat, hydriertes Xylylen-diisocyanat (H6XDI), 1-Methyl-2,4-diisocyanatocyclohexan, Tetramethoxybutan-1,4-diisocyanat, Butan-1,4-diisocyanat, 1,6-Diisocyanato-2,2,4-trimethylhexan, 1,6-Diisocyanato-2,4,4-trimethylhexan, 1-Isocyanato-1-methyl-4(3)-isocyanatomethylcyclohexan (IMCI) sowie 1,12-Dodecandiisocyanat (C12DI) aus dem Umsetzungsprodukt entfernt wird.

Bei den gegenüber Isocyanaten reaktiven Verbindungen handelt es sich bevorzugt um Hydroxylgruppen und/oder Aminogruppen aufweisende Verbindungen. Besonders bevorzugt sind Polyetherpolyole, Polyesterpolyole, Polybutadienepolyole und Polycarbonatpolyole. Die Polyole und/oder Polyamine enthalten bevorzugt zwischen zwei und 10, besonders bevorzugt zwischen zwei und drei Hydroxylgruppen und/oder Aminogruppen und besitzen ein gewichtsmittleres Molekulargewicht zwischen 32 und 20000, besonders bevorzugt zwischen 90 und 18000 g/mol. Als Polyole eignen sich vorzugsweise die bei Raumtemperatur flüssigen, glasartig fest/amorphen oder kristallinen Polyhydroxyverbindungen. Als typische Beispiele wären difunktionelle Polypropylenglykole zu nennen. Es können auch Hydroxylgruppen aufweisende statistische Copolymere und/oder Blockcopolymere des Ethylenoxids und Propylenoxids eingesetzt werden. Geeignete Polyetherpolyole sind die in der Polyurethanchemie an sich bekannten Polyether, wie die unter Verwendung von Startermolekülen hergestellten Polyole aus Styroloxid, Propylenoxid, Butylenoxid, Tetrahydrofuran oder Epichlorhydrin. Konkret eignen sich insbesondere auch Poly(oxytetramethylen)glykol (Poly-THF), 1,2-Polybutylenglykol, oder deren Mischungen. Insbesondere geeignet sind Polypropylenoxid und Polyethylenoxid und deren Mischungen. Ein weiterer als Polyolkomponente einsetzbarer Copolymertyp, der endständig Hydroxylgruppen aufweist, ist gemäß der allgemeinen Formel (herstellbar z.B. mittels " Controlled" High-Speed Anionic Polymerization gemäß Macromolecules 2004, 37, 4038-4043): 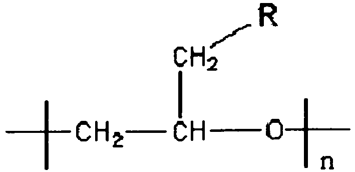 in welcher R gleich oder verschieden ist und bevorzugt durch OMe, OiPr, Cl oder Br repräsentiert wird.

Weiterhin eignen sich als Polyolkomponente die bei 25°C flüssigen, glasartig amorphen oder kristallinen Polyesterdi- bzw. polyole, die durch Kondensation von Di- oder Tricarbonsäuren, wie Adipinsäure, Sebacinsäure, Glutarsäure, Azelainsäure, Korksäure, Undecandisäure, Dodecandisäure, 3,3-Dimethylglutarsäure, Terephthalsäure, Isophthalsäure, Hexahydrophthalsäure und/oder Dimerfettsäure, mit niedermolekularen Diolen bzw. Triolen, wie Ethylenglykol, Propylenglykol, Diethylenglykol, Triethylenglykol, Dipropylenglykol, 1,4-Butandiol, 1,6-Hexandiol, 1,8-Octandiol, 1,10-Decandiol, 1,12-dodecandiol, Dimerfettalkohol, Glycerin und/ oder Trimethylolpropan, herstellbar sind.

Eine weitere geeignete Gruppe der Polyole sind die Polyester z.B. auf der Basis von Caprolacton, welche auch als "Polycaprolactone" bezeichnet werden. Weitere einsetzbare Polyole sind Polycarbonat-Polyole und Dimerdiole sowie Polyole auf Basis pflanzlicher Öle und ihrer Derivate, wie Rizinusöl und dessen Derivate, oder epoxidiertes Sojabohnenöl. Außerdem kommen Hydroxylgruppen aufweisende Polycarbonate in Frage, welche durch Reaktion von Kohlensäurederivaten, z. B. Diphenylcarbonat, Dimethylcarbonat oder Phosgen, mit Diolen erhältlich sind. Konkret eignen sich Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A, Tetrabrombisphenol A, Glyzerin, Trimethylolpropan, Hexantriol-1,2,6, Butantriol-1,2,4, Trimethylolpropan, Pentaerythrit, Chinit, Mannit, Sorbit, Methylglykosid und 1,3,4,6-Dianhydrohexite. Auch die Hydroxy-funktionellen Polybutadiene, welche u.a. unter dem Handelsnamen "Poly-bd®" käuflich sind, können als Polyole ebenso wie deren hydrierten Analoga eingesetzt werden. Weiterhin kommen Hydroxy-funktionelle Polysulfide, welche z. B. unter dem Handelsnamen "Thiokol® NPS-282" vertrieben werden, sowie Hydroxy-funktionelle Polysiloxane in Frage.

Als erfindungsgemäß einsetzbare Polyamine eignen sich insbesondere Hydrazin, Hydrazinhydrat und substituierte Hydrazine, wie N-Methylhydrazin, N,N'-Dimethylhydrazin, Säuredihydrazide, Adipinsäure, Methyladipinsäure, Sebacinsäure, Hydracrylsäure, Terephthalsäure, Semicarbazidoalkylen-hydrazide, wie 13-Semicarbazidopropionsäurehydrazid, Semicarbazidoalkylen-carbazinester, wie z. B. 2-Semicarbazidoethyl-carbazinester und/ oder Aminosemicarbazid-Verbindungen, wie 13-Aminoethylsemicarbazidocarbonat.

Polyamine, z.B. solche, die unter dem Handelsnamen Jeffamine® (es handelt sich um Polyetherpolyamine) vertrieben werden, sind auch geeignet.

Als Polyole und/oder Polyamine kommen auch die als sogenannte Kettenverlänger bekannten Spezies in Frage, welche bei der Herstellung von Polyurethanen und Polyharnstoffen mit überschüssigen Isocyanatgruppen reagieren, normalerweise ein Molekulargewicht von unter 400 aufweisen und häufig in Form von Polyolen, Aminopolyolen oder aliphatischen, cycloaliphatischen oder araliphatischen Polyaminen vorliegen.

Geeignete Kettenverlängerer sind beispielsweise:
- Alkandiole, wie Ethandiol, 1,2- und 1,3-Propandiol, 1,4- und 2,3-Butandiol, 1,5-Pentandiol, 1,3-Dimethylpropandiol, 1,6-Hexandiol, Neopentylglykol, Cyclohexandimethanol, 2-Methyl-1,3-propandiol,
- Etherdiole, wie Diethylendiglykol, Triethylenglykol oder Hydrochinondihydroxyethylether
- Hydroxybutylhydroxycapronsäureester, Hydroxyhexylhydroxybuttersäureester, Adipinsäurehydroxyethylester und Terephthalsäurebishydroxyethylester und
- Polyamine, wie Ethylendiamin, 1,2- und 1,3-Diaminopropan, 1,4-Diaminobutan, 1,6-Diaminohexan, Isomerengemisch von 2,2,4- und 2,4,4-Trimethylhexamethylendiamin, 2-Methyl-pentamethylendiamin, Diethylentriamin, 1,3- sowie 1,4-Xylylendiamin und 4,4- Diaminodicyclohexylmethan

Schließlich soll erwähnt sein, dass die Polyole und/oder Polyamine Doppelbindungen enthalten können, welche z.B. aus langkettigen, aliphatischen Carbonsäuren oder Fettalkoholen resultieren können. Eine Funktionalisierung mit olefinischen Doppelbindungen ist z. B. auch durch den Einbau vinylischer oder allylischer Gruppen möglich. Diese können beispielsweise von ungesättigten Säuren wie Maleinsäureanhydrid, Acrylsäure oder Methacrylsäure sowie deren jeweiligen Estern stammen.

Als besonders bevorzugt im Sinne der Erfindung sind Umsetzungsprodukt welche auf Polypropylendiol, Polypropylentriol, Polypropylenpolyol, Polyethylendiol, Polyethylentriol, Polyethylenpolyol, Polypropylendiamin, Polypropylentriamin, Polypropylenpolyamin, Poly-THF-diamin, Polybutadiendiol, Polyesterdiol, Polyestertriol, Polyesterpolyol, Polyesteretherdiol, Polyesterethertriol, Polyesteretherpolyol, besonders bevorzugt Polypropylendiol, Polypropylentriol, Poly-THF-diol, Polyhexandiolcarbamatdiol, Polycaprolactamdiol, Polycaprolactamtriol und Wasser als gegenüber Isocyanaten reaktive Verbindung basieren. Weiterhin kann es sich auch um Mischungen der genannten Verbindungen handeln.

Es hat sich in diesem Zusammenhang als besonders überraschend erwiesen, dass das erfindungsgemäße Verfahren sich in hervorragender Weise auch für die Entfernung von Isocyanat aus hochviskosen Flüssigkeiten eignet, wobei auch kleinste Mengen an Isocyanat effektiv entfernt werden können. Neben der Viskosität spielen auch die chemischen Eigenschaften der eingesetzten Umsetzungsprodukte eine wichtige Rolle. Das Verfahren gemäß vorliegender Erfindung liefert sowohl für Polyurethane als auch Polyharnstoffe sowie für oligomere Isocyanatgemische hervorragende Ergebnisse. Weiterhin ist das erfindungsgemäße Verfahren mit geringem apparativem Aufwand durchführbar, wobei relativ hohe Stoffdurchsätze möglich sind. Somit liefert das beanspruchte Verfahren auch für die großtechnische Aufreinigung von Umsetzungsprodukten des Isocyanates eine sehr kostengünstige Alternative zu den bereits bekannten Verfahren.

Eine besondere Ausführungsform der vorliegenden Erfindung sieht vor, dass man ein Umsetzungsprodukt einsetzt, welches hergestellt wurde durch Umsetzung von Isocyanat mit gegenüber Isocyanaten reaktiven Verbindungen in einem Reaktor, welcher aufweist
- α): einen um eine bevorzugt zentral angeordnete Rotationsachse rotierenden heißen Körper B,
- β): ein Dosierungssystem und
- γ): eine Quenscheinrichtung,
wobei
a) das Isocyanat und die gegenüber Isocyanaten reaktiven Verbindungen einzeln und/oder als Mischung, gegebenenfalls mit weiteren Komponenten, mit Hilfe des Dosierungssystems auf die Oberfläche des rotierenden Körpers B aufgetragen werden, so dass ein Film enthaltend gegenüber Isocyanaten reaktive Verbindungen und Isocyanat über die Oberfläche des rotierenden Körpers B zu einem äußeren Bereich der heißen Oberfläche des rotierenden Körpers B fließt,
b) der Film die Oberfläche als Polyurethan und/oder Polyharnstoff enthaltendes Umsetzungsprodukt verlässt und
c) die Reaktionskomposition mittels der Quenscheinrichtung nach Verlassen der heißen Oberfläche abrupt abgekühlt wird,
die Temperatur der Oberfläche des rotierenden Körpers B zwischen 70 bis 300 °C, besonders bevorzugt zwischen 160 und 250 °C beträgt und die mittels der Quenscheinrichtung erfolgte abrupte Abkühlung der Reaktionskomposition mindestens 30 °C beträgt.

Die Quenscheinrichtung liegt im Allgemeinen bevorzugt in Form einer oder mehrerer Kühlwände vor, welche die abrupte Abkühlung des Reaktionsgemischs ermöglicht. Die Kühlwände, welche häufig zylinder- oder kegelförmig sind, weisen entweder eine glatte oder eine raue Oberfläche auf, deren Temperatur typischerweise zwischen -50 °C und 200 °C liegt. Die mittels der Quenscheinrichtung erfolgte abrupte Abkühlung der Reaktionskomposition beträgt bevorzugt mindestens 50 °C, bevorzugt mindestens 100 °C.

Der heiße rotierende Körper B ist hierbei zweckmäßigerweise entsprechend dem Körper A beschaffen. Insbesondere ist es hierbei von Vorteil, dass sowohl die Herstellung des Umsetzungsproduktes von Isocyanat mit gegenüber Isocyanaten reaktiven Verbindungen als auch die Entfernung des Isocyanates aus dem Umsetzungsprodukt mit der gleichen Apparatur durchgeführt werden kann.

Das molare Verhältnis der Isocyanatgruppen der eingesetzten Isocyanatkomponente zu der Summe der Aminogruppen und/oder Hydroxylgruppen der eingesetzten Polyole und/oder Polyamine liegt bevorzugt zwischen 0,1 und 20, besonders bevorzugt zwischen 1,3 und 10, insbesondere zwischen 1,8 und 5.

Vorteilhaft wird in dem erfindungsgemäßen Verfahren ein für die Herstellung von Polyurethanen oder Polyharnstoffen geeigneter Katalysator als Komponente der Ausgangsreaktionsmischung eingesetzt. Als Katalysatoren geeignet sind die an sich bekannten, üblichen Katalysatoren der Polyurethanchemie, wie Säuren, z. B. para-Toluolsulfonsäure oder tert. Amine, wie z. B. Triethylamin, Triethylendiamin (DABCO) oder solche welche Atome, wie z.B. Sn, Mn, Fe, Co, Cd, Ni, Cu, Zn, Zr, Ti, Hf, Al, Th, Ce, Bi, Hg, N, P, aufweisen. Das molare Verhältnis Katalysator / Isocyanat ist abhängig vom Typ des Isocyanates und der Art des Katalysators und liegt normalerweise zwischen von 0 bis 0,1, bevorzugt 0 bis 0,03.

Die Temperatur des heißen Körpers B und die Kontaktzeit auf diesem Körper wird bevorzugt so eingestellt, dass zwischen 5 und 99,99 Gew.-% der maximal mit der eingesetzten Menge an Polyol und gegebenenfalls Amin umsetzbaren Isocyanatgruppen, bevorzugt mit Hydroxyl- und gegebenenfalls Amingruppen des Polyols und gegebenenfalls Amins reagiert haben.

### Beispiele

Nachfolgende Beispiele wurden auf einem rotierenden Körper A durchgeführt, der als eine glatte Scheibe (bestehend aus Kupfer mit verchromter Oberfläche) mit einem Durchmesser von 20 cm vorlag, welche beheizt wurde, und sich auf einer vertikalen Achse befand. Ähnliche Reaktoren werden auch in den Dokumenten WO00/48728, WO00/48729, WO00/48730, WO00/48731 und WO00/48732 näher beschrieben. Die Scheibe war von einem metallischen Gehäuse umgeben, an dem ein Vakuum angelegt werden kann. Das Produkt wurde in einem angeschraubten, geschlossenen Gefäß aufgefangen.

### Beispiel 1: Herstellung eines Polyurethan-Präpolymers mit 5-fachem Überschuss an Isophorondiisocyanat

PPG 2000 (Arcol® PPG-2000, Fa Bayer) wurde mit 233,4 g Isophorondiisocyanat (IP-DI) (NCO/OH-Verhältnis 6 : 1) vermischt und bei 50°C mit 60 mg Dibutylzinndilaurat (DBTL) versetzt. Nach 3 h betrug der NCO-Gehalt, der nach Probenentnahme in dem Gemisch gemessen wurde, nur noch 12,6 Gew.-%. Das klare, farblose Produkt wurde abgekühlt und für Beispiel 2 verwendet. Der IPDI-Gehalt wurde mittels GC bestimmt und betrug 26,7 Gew.-%.

### Beispiel 2: Entfernung von Isocyanatverbindungen aus einem Polyurethan-Präpolymer (Beispiel 1) im Vakuum

Ein Polyurethan-Präpolymer (Beispiel 1) mit einem Rest-Monomergehalt an IPDI von 26,7 Gew.-% und einer Viskosität von 1040 mPa s (23°C, nach Brookfield) sowie einem Anfangs-NCO-Gehalt von 12,6 Gew.-% wird auf die Mitte der Oberfläche einer 200°C heißen, rotierenden Scheibe aufgetragen. Die Scheibe rotiert mit 2000 U /min. Bei ca. 8 mbar wird das Präpolymer mit einer Zahnradpumpe mit 1 ml/s auf die Scheibe dosiert und fließt aufgrund der Zentrifugalkräfte als Film nach außen. Das Präpolymer fließt nach Verlassen der Scheibe an der senkrecht dazu stehenden Gehäusewand herunter und wird gesammelt. Es verlässt das System mit ca. 90°C. Der NCO-Gehalt des gesammelten Präpolymers beträgt nur noch 9,8 Gew.-%. Der Monomergehalt (IPDI) beträgt 19,5 Gew.-%. Die Molekulargewichtsverteilung der Polymeranteile (ermittelt durch GPC) bleibt unverändert.

### Beispiel 3: Entfernung von Isocyanatverbindungen aus einem käuflichen Polyurethan-Präpolymer im Vakuum

Ein Polyurethan-Präpolymer (Conipur M 865 Z, basierend auf Isophorondiisocyanat (IPDI)) mit einer Viskosität von ca. 5450 mPa s, einem NCO-Gehalt von 3.9 Gew.-% und einem Monomergehalt (IPDI) von 2,6 Gew.-% wird auf die Mitte der Oberfläche eines 200°C heissen rotierenden Körpers A aufgetragen. Die Scheibe rotiert mit 2000 U/min. Bei ca. 4 mbar wird das Präpolymer mit einer Zahnradpumpe mit 1 ml / s auf die Scheibe dosiert und fließt aufgrund der Zentrifugalkräfte als Film nach außen. Das Präpolymer fließt nach Verlassen der Scheibe an der senkrecht dazu stehenden Gehäusewand herunter und wird gesammelt. Es verlässt das System mit ca. 90°C. Der NCO-Gehalt des gesammelten Präpolymers beträgt nur noch 3,5 Gew.-%. Der Monomergehalt (IPDI) beträgt nun nur noch 1,7 Gew.-%.

## Patentansprüche

1. Verfahren zur Entfernung von Isocyanat aus einem Umsetzungsprodukt von Isocyanat mit gegenüber Isocyanaten reaktiven Verbindungen, **dadurch gekennzeichnet, dass** man das Umsetzungsprodukt auf die Oberfläche eines rotierenden Körpers A aufträgt, wobei das Umsetzungsprodukt über die Oberfläche des rotierenden Körpers A zu einem äußeren Bereich der Oberfläche des rotierenden Körpers A fließt und dabei Isocyanat, das zur Herstellung des Umsetzungsproduktes eingesetzt und nicht umgesetzt wurde, aus der Mischung verdampft.

2. Verfahren nach Anspruch 1, **dadurch gekennzeichnet, dass** der rotierende Körper A als eine Drehscheibe vorliegt, auf dessen Oberfläche das Umsetzungsprodukt aufgetragen wird.

3. Verfahren nach Anspruch 1 oder 2, **dadurch gekennzeichnet, dass** mandie Entfernung des Isocyanates mittels einer Apparatur durchführt, die
α) einen um eine Rotationsachse rotierenden Körper A und
β) ein Dosierungssystem,
aufweist.

4. Verfahren nach einem der Ansprüche 1 bis 3, **dadurch gekennzeichnet, dass** das Umsetzungsprodukt auf der Oberfläche eines rotierenden Körpers A in Form eines Films vorliegt, der eine durchschnittliche Dicke zwischen 0,1 µm bis 6,0 mm aufweist.

5. Verfahren nach einem der Ansprüche 1 bis 4, **dadurch gekennzeichnet, dass** die durchschnittliche Verweilzeit der Inhaltsstoffe des Umsetzungsproduktes auf der Oberfläche des rotierenden Körpers zwischen 0,01 und 60 Sekunden beträgt.

6. Verfahren nach einem der Ansprüche 1 bis 5, **dadurch gekennzeichnet, dass** die Temperatur des rotierenden Körpers zwischen 70 und 300 °C beträgt.

7. Verfahren nach einem der Ansprüche 1 bis 6, **dadurch gekennzeichnet, dass** der Druck, bei dem das Isocyanat entfernt wird, zwischen 0,001 mbar und 1100 mbar beträgt.

8. Verfahren nach einem der Ansprüche 1 bis 7, **dadurch gekennzeichnet, dass** man das verdampfte Isocyanat an einem Körper mit einer Temperatur zwischen -196 °C und 120 °C kondensiert.

9. Verfahren nach einem der Ansprüche 1 bis 8, **dadurch gekennzeichnet, dass** direkt vor dem Auftragen des Substrats auf die Oberfläche des rotierenden Körpers A der Gehalt an Isocyanat zwischen 0,01 und 95 Gew.-% beträgt, bezogen auf das Gesamtgewicht des Umsetzungsproduktes.

10. Verfahren nach einem der Ansprüche 1 bis 9, **dadurch gekennzeichnet, dass** der Gehalt an Isocyanat in dem Umsetzungsprodukt nach dem Verdampfen des Isocyanates auf der Oberfläche eines rotierenden Körpers A zwischen 0,001 und 10 Gew.-% beträgt, bezogen auf das Gesamtgewicht des Umsetzungsproduktes.

11. Verfahren nach einem der Ansprüche 1 bis 10, **dadurch gekennzeichnet, dass** das Umsetzungsprodukt basiert auf der Umsetzung von Hexamethylendiisocyanat-1,6 (HDI), 1-Isocyanato-3,3,5-trimethyl-5-isocyanatomethylcyclohexan (IP-DI), 2,4- und/oder 2,6-Toluylendiisocyanat (TDI) und/oder 4,4' -, 2,4' - und/oder 2,2' -Diphenylmethandiisocyanat (MDI), m-Xylendiisocyanat (MXDI), m- oder p-Tetramethylxylendiisocyanat (m-TMXDI, p-TMXDI), 4,4'-Dicyclohexylmethandiisocyanat (H12MDI), Naphthalin-1,5-Diisocyanat, Cyclohexan-1,4-diisocyanat, hydriertes Xylylen-diisocyanat (H6XDI), 1-Methyl-2,4-diisocyanato-cyclohexan, Tetramethoxybutan-1,4-diisocyanat, Butan-1,4-diisocyanat, 1,6-Diisocyanato-2,2,4-trimethylhexan, 1,6-Diisocyanato-2,4,4-trimethylhexan, 1-Isocyanato-1-methyl-4(3)-isocyanatomethylcyclohexan (IMCI) sowie 1,12-Dodecandiisocyanat (C12DI) mit gegenüber Isocyanaten reaktiven Verbindungen.

12. Verfahren nach einem der Ansprüche 1 bis 11, **dadurch gekennzeichnet, dass** man Hexamethylendiisocyanat-1,6-(HDI), 1-Isocyanato-3,3,5-trimethyl-5-isocyanatomethylcyclohexan (IPCI), 2,4- und/oder 2,6-Toluylendiisocyanat (TDI) und/oder 4,4' -, 2,4' - und/oder 2,2' -Diphenylmethandiisocyanat (MDI), m-Xylendiisocyanat (MXDI), m- oder p-Tetramethylxylendiisocyanat (m-TMXDI, p-TMXDI), 4,4'-Dicyclohexylmethandiisocyanat (H12MDI), Naphthalin-1,5-Diisocyanat, Cyclohexan-1,4-diisocyanat, hydriertes Xylylen-diisocyanat (H6XDI), 1-Methyl-2,4-diisocyanato-cyclohexan, Tetramethoxybutan-1,4-diisocyanat, Butan-1,4-diisocyanat, 1,6-Diisocyanato-2,2,4-trimethylhexan, 1,6-Diisocyanato-2,4,4-trimethylhexan, 1-Isocyanato-1-methyl-4(3)-isocyanatomethylcyclohexan (IMCI) sowie 1,12-Dodecandiisocyanat (C12DI) aus dem Umsetzungsprodukt entfernt.

13. Verfahren nach einem der Ansprüche 1 bis 12, **dadurch gekennzeichnet, dass** das Umsetzungsprodukt basiert auf Polypropylendiol, Polypropylentriol, Polypropylenpolyol, Polyethylendiol, Polyethylentriol, Polyethylenpolyol, Polypropylendiamin, Polypropylentriamin, Polypropylenpolyamin, Poly-THF-diamin, Polybutadiendiol, Polyesterdiol, Polyestertriol, Polyesterpolyol, Polyesteretherdiol, Polyesterethertriol, Polyesteretherpolyol, Polypropylendiol, Polypropylentriol, Poly-THF-diol, Polyhexandiolcarbamatdiol, Polycaprolactamdiol, Polycaprolactamtriol und Wasser als gegenüber Isocyanaten reaktive Verbindungen.

14. Verfahren nach einem der Ansprüche 1 bis 13, **dadurch gekennzeichnet, dass** man ein Umsetzungsprodukt einsetzt, welches hergestellt wurde durch Umsetzung von isocyanat mit gegenüber Isocyanaten reaktiven Verbindungen in einem Reaktor, welcher aufweist
α) einen um eine Rotationsachse rotierenden heißen Körper B,
β) ein Dosierungssystem und
γ) eine Quenscheinrichtung,
wobei
a) das Isocyanat und die gegenüber Isocyanaten reaktiven Verbindungen einzeln und/oder als Mischung, gegebenenfalls mit weiteren Komponenten, mit Hilfe des Dosierungssystems auf die Oberfläche des rotierenden Körpers B aufgetragen werden, so dass ein Film enthaltend gegenüber Isocyanaten reaktive Verbindungen und Isocyanat über die Oberfläche des rotierenden Körpers B zu einem äußeren Bereich der heißen Oberfläche des rotierenden Körpers B fließt,
b) der Film die Oberfläche als Polyurethan und/oder Polyharnstoff enthaltendes Umsetzungsprodukt verlässt und
c) die Reaktionskomposition mittels der Quenscheinrichtung nach Verlassen der heißen Oberfläche abrupt abgekühlt wird,
die Temperatur der Oberfläche des rotierenden Körpers B zwischen 70 und 300 °C beträgt und die mittels der Quenscheinrichtung erfolgte abrupte Abkühlung der Reaktionskomposition mindestens 30 °C beträgt.

## Claims

1. Method for removing isocyanate from a reaction product of isocyanate with compounds reactive towards isocyanates, **characterized in that** the reaction product is applied to the surface of a rotating body A, the reaction product flowing over the surface of the rotating body A to an outer region of the surface of the rotating body A and isocyanate which was used for the preparation of the reaction product and has not reacted evaporating from the mixture in the process.

2. Method according to Claim 1, **characterized in that** the rotating body A is present as a rotating disc, to the surface of which the reaction product is applied.

3. Method according to Claim 1 or 2, **characterized in that** the removal of the isocyanate is carried out by means of an apparatus which has
α) a body A rotating about an axis of rotation and
β) a metering system.

4. Method according to any of Claims 1 to 3, **characterized in that** the reaction product is present on the surface of a rotating body A in the form of a film which has an average thickness between 0.1 µm and 6.0 mm.

5. Method according to any of Claims 1 to 4, **characterized in that** the average residence time of the ingredients of the reaction product on the surface of the rotating body is between 0.01 and 60 seconds.

6. Method according to any of Claims 1 to 5, **characterized in that** the temperature of the rotating body is between 70 and 300°C.

7. Method according to any of Claims 1 to 6, **characterized in that** the pressure at which the isocyanate is removed is between 0.001 mbar and 1100 mbar.

8. Method according to any of Claims 1 to 7, **characterized in that** the evaporated isocyanate condenses on a body having a temperature between -196°C and 120°C.

9. Method according to any of Claims 1 to 8, **characterized in that** the content of isocyanate is between 0.01 and 95% by weight, based on the total weight of the reaction product, directly before the application of the substrate to the surface of the rotating body A.

10. Method according to any of Claims 1 to 9, **characterized in that** the content of isocyanate in the reaction product is between 0.001 and 10% by weight, based on the total weight of the reaction product, after the evaporation of the isocyanate on the surface of a rotating body A.

11. Method according to any of Claims 1 to 10, **characterized in that** the reaction product is based on the reaction of hexamethylene 1,6-diisocyanate (HDI), 1-isocyanato-3,3,5-trimethyl-5-isocyanatomethylcyclohexane (IPDI), 2,4- and/or 2,6-toluylene diisocyanate (TDI) and/or 4,4'-, 2,4'- and/or 2,2'-diphenylmethane diisocyanate (MDI), m-xylene diisocyanate (MXDI), m- or p-tetramethylxylene diisocyanate (m-TMXDI, p-TMXDI), 4,4'-dicyclohexylmethane diisocyanate (H12MDI), naphthalene 1,5-diisocyanate, cyclohexane 1,4-diisocyanate, hydrogenated xylylene diisocyanate (H6XDI), 1-methyl-2,4-diisocyanatocyclohexane, tetramethoxybutane 1,4-diisocyanate, butane 1,4-diisocyanate, 1,6-diisocyanato-2,2,4-trimethylhexane, 1,6-diisocyanato-2,4,4-trimethylhexane, 1-isocyanato-1-methyl-4(3)-isocyanatomethylcyclohexane (IMCI) and 1,12-dodecane diisocyanate (C12DI) with compounds reactive towards isocyanates.

12. Method according to any of Claims 1 to 11, **characterized in that** hexamethylene 1,6-diisocyanate (HDI), 1-isocyanato-3,3,5-trimethyl-5-isocyanatomethylcyclohexane (IPDI), 2,4- and/or 2,6-toluylene diisocyanate (TDI) and/or 4,4'-, 2,4'- and/or 2,2'-diphenylmethane diisocyanate (MDI), m-xylene diisocyanate (MXDI), m- or p-tetramethylxylene diisocyanate (m-TMXDI, p-TMXDI), 4,4'-dicyclohexylmethane diisocyanate (H12MDI), naphthalene 1,5-diisocyanate, cyclohexane 1,4-diisocyanate, hydrogenated xylylene diisocyanate (H6XDI), 1-methyl-2,4-diisocyanatocyclohexane, tetramethoxybutane 1,4-diisocyanate, butane 1,4-diisocyanate, 1,6-diisocyanato-2,2,4-trimethylhexane, 1,6-diisocyanato-2,4,4-trimethylhexane, 1-isocyanato-1-methyl-4(3)-isocyanatomethylcyclohexane (IMCI) and 1,12-dodecane diisocyanate (C12DI) are removed from the reaction product.

13. Method according to any of Claims 1 to 12, **characterized in that** the reaction product is based on polypropylenediol, polypropylenetriol, polypropylenepolyol, polyethylenediol, polyethylenetriol, polyethylenepolyol, polypropylenediamine, polypropylenetriamine, polypropylenepolyamine, poly-THF-diamine, polybutadienediol, polyesterdiol, polyestertriol, polyesterpolyol, polyesteretherdiol, polyesterethertriol, polyesteretherpolyol, polypropylenediol, polypropylenetriol, poly-THF-diol, polyhexanediol carbamate diol, polycaprolactamdiol, polycaprolactamtriol and water as compounds reactive towards isocyanates.

14. Method according to any of Claims 1 to 13, **characterized in that** a reaction product is used which was prepared by reacting isocyanate with compounds reactive towards isocyanates in a reactor which has
α) a hot body B rotating about an axis of rotation,
β) a metering system and
γ) a quench device,
a) the isocyanate and the compounds reactive towards isocyanates being applied individually and/or as a mixture, optionally with further components, with the aid of the metering system to the surface of the rotating body B so that a film containing compounds reactive towards isocyanates and isocyanate flows over the surface of the rotating body B to an outer region of the hot surface of the rotating body B,
b) the film leaving the surface as a reaction product containing polyurethane and/or polyurea and
c) the reaction composition being cooled abruptly by means of the quench device after leaving the hot surface,
the temperature of the surface of the rotating body B being between 70 and 300°C and the abrupt cooling of the reaction composition effected by means of the quench device being at least 30°C.

## Revendications

1. Procédé pour l'élimination d'isocyanate d'un produit de transformation d'isocyanate avec des composés réactifs vis-à-vis d'isocyanates, **caractérisé en ce qu'**on applique le produit de transformation sur la surface d'un corps A en rotation, le produit de transformation s'écoulant sur la surface du corps A en rotation vers une zone externe de la surface du corps A en rotation et l'isocyanate qui a été utilisé pour la préparation du produit de transformation et qui n'a pas été transformé, s'évaporant du mélange.

2. Procédé selon la revendication 1, **caractérisé en ce que** le corps A en rotation se trouve sous forme d'un disque rotatif, à la surface duquel est appliqué le produit de transformation.

3. Procédé selon la revendication 1 ou 2, **caractérisé en ce qu'**on réalise l'élimination de l'isocyanate au moyen d'un appareil, qui présente
α) un corps A tournant autour d'un axe de rotation et
β) un système de dosage.

4. Procédé selon l'une quelconque des revendications 1 à 3, **caractérisé en ce que** le produit de transformation se trouve sur la surface d'un corps A en rotation sous forme d'un film qui présente une épaisseur moyenne entre 0,1 µm à 6,0 mm.

5. Procédé selon l'une quelconque des revendications 1 à 4, **caractérisé en ce que** le temps de séjour moyen des constituants du produit de transformation à la surface du corps en rotation est situé entre 0,01 et 60 secondes.

6. Procédé selon l'une quelconque des revendications 1 à 5, **caractérisé en ce que** la température du corps en rotation est située entre 70 et 300°C.

7. Procédé selon l'une quelconque des revendications 1 à 6, **caractérisé en ce que** la pression à laquelle l'isocyanate est éliminé est située entre 0,001 mbar et 1100 mbars.

8. Procédé selon l'une quelconque des revendications 1 à 7, **caractérisé en ce qu'**on condense l'isocyanate évaporé sur un corps présentant une température entre -196°C et 120°C.

9. Procédé selon l'une quelconque des revendications 1 à 8, **caractérisé en ce que** juste avant l'application du substrat sur la surface du corps A en rotation, la teneur en isocyanate est située entre 0,01 et 95% en poids, par rapport au poids total du produit de transformation.

10. Procédé selon l'une quelconque des revendications 1 à 9, **caractérisé en ce que** la teneur en isocyanate dans le produit de transformation après l'évaporation de l'isocyanate à la surface d'un corps A en rotation est située entre 0,001 et 10% en poids, par rapport au poids total du produit de transformation.

11. Procédé selon l'une quelconque des revendications 1 à 10, **caractérisé en ce que** le produit de transformation est à base de la transformation d'hexaméthylènediisocyanate-1,6 (HDI), de 1-isocyanato-3,3,5-triméthyl-5-isocyanatométhylcyclohexane (IPDI), de 2,4-toluylènediisocyanate et/ou de 2,6-toluylènediisocyanate (TDI) et/ou de 4,4'-diphénylméthanediisocyanate, de 2,4'-diphénylméthanediisocyanate et/ou de 2,2'-diphénylméthanediisocyanate (MDI), de m-xylènediisocyanate (MXDI), de m-tétraméthylxylènediisocyanate ou de p-tétraméthylxylènediisocyanate (m-TMXDI, p-TMXDI), de 4,4'-dicyclohexylméthanediisocyanate (H12MDI), de naphtalène-1,5-diisocyanate, de cyclohexane-1,4-diisocyanate, de xylylènediisocyanate hydrogéné (H6XDI), de 1-méthyl-2,4-diisocyanatocyclohexane, de tétraméthoxybutane-1,4-diisocyanate, de butane-1,4-diisocyanate, de 1,6-diisocyanato-2,2,4-triméthylhexane, de 1,6-diisocyanato-2,4,4-triméthylhexane, de 1-isocyanato-1-méthyl-4(3)-isocyanatométhylcyclohexane (IMCI) ainsi que de 1,12-dodécanediisocyanate (C12DI) avec des composés réactifs vis-à-vis d'isocyanate.

12. Procédé selon l'une quelconque des revendications 1 à 11, **caractérisé en ce qu'**on élimine l'hexaméthylènediisocyanate-1,6 (HDI), le 1-isocyanato-3,3,5-triméthyl-5-isocyanatométhylcyclohexane (IPDI), le 2,4-toluylènediisocyanate et/ou le 2,6-toluylènediisocyanate (TDI) et/ou le 4,4'-diphénylméthanediisocyanate, le 2,4'-diphénylméthanediisocyanate et/ou le 2,2'-diphénylméthanediisocyanate (MDI), le m-xylènediisocyanate (MXDI), le m-tétraméthylxylènediisocyanate ou le p-tétraméthylxylènediisocyanate (m-TMXDI, p-TMXDI), le 4,4'-dicyclohexylméthanediisocyanate (H12MDI), le naphtalène-1,5-diisocyanate, le cyclohexane-1,4-diisocyanate, le xylylènediisocyanate hydrogéné (H6XDI), le 1-méthyl-2,4-diisocyanatocyclohexane, le tétraméthoxybutane-1,4-diisocyanate, le butane-1,4-diisocyanate, le 1,6-diisocyanato-2,2,4-triméthylhexane, le 1,6-diisocyanato-2,4,4-triméthylhexane, le 1-isocyanato-1-méthyl-4(3)-isocyanatométhylcyclohexane (IMCI) ainsi que le 1,12-dodécanediisocyanate (C12DI) du produit de transformation.

13. Procédé selon l'une quelconque des revendications 1 à 12, **caractérisé en ce que** le produit de transformation est à base de polypropylènediol, de polypropylènetriol, de polypropylènepolyol, de polyéthylènediol, de polyéthylènetriol, de polyéthylènepolyol, de polypropylènediamine, de polypropylènetriamine, de polypropylènepolyamine, de poly-THF-diamine, de polybutadiènediol, de polyesterdiol, de polyestertriol, de polyesterpolyol, de polyesterétherdiol, de polyesteréthertriol, de polyesterétherpolyol, de polypropylènediol, de polypropylènetriol, de poly-THF-diol, de polyhexanediolcarbamatediol, de polycaprolactamediol, de polycaprolactametriol et d'eau comme composés réactifs vis-à-vis d'isocyanate.

14. Procédé selon l'une quelconque des revendications 1 à 13, **caractérisé en ce qu'**on utilise un produit de transformation, qui a été préparé par la transformation d'isocyanate avec des composés réactifs vis-à-vis d'isocyanate dans un réacteur, qui présente
α) un corps B chaud tournant autour d'un axe de rotation
β) un système de dosage et
γ) un dispositif de refroidissement brusque,
où
a) l'isocyanate et les composés réactifs vis-à-vis d'isocyanate sont appliqués seuls et/ou sous forme de mélange, le cas échéant avec d'autres composants, à l'aide du système de dosage à la surface du corps B en rotation, de manière telle qu'un film contenant des composés réactifs vis-à-vis d'isocyanate et l'isocyanate s'écoule sur la surface du corps B en rotation, vers une zone extérieure de la surface chaude du corps B en rotation,
b) le film quitte la surface sous forme de produit de transformation contenant du polyuréthane et/ou de la polyurée et
c) la composition de réaction est refroidie brusquement au moyen du dispositif de refroidissement brusque après avoir quitté la surface chaude,
la température de la surface du corps B en rotation étant située entre 70 et 300°C et le refroidissement brusque réalisé à l'aide du dispositif de refroidissement brusque de la composition de réaction étant d'au moins 30°C.
